# EUROPEAN PATENT SPECIFICATION

(11) **EP 4 440 339 B1**
(45) Date of publication and mention of the grant of the patent: **15.10.2025**
(21) Application number: 22823566.9
(22) Date of filing: 02.12.2022
(51) Int. Cl.: A23L 33/135, A23L 33/19, A23L 33/21, A61K 31/702, A61P 1/00

(54) **NUTRITIONAL COMPOSITION FOR IMPROVING INFANT MICROBIOTA**
ERNÄHRUNGSZUSAMMENSETZUNG ZUR VERBESSERUNG VON KLEINKINDMIKROBIOTA
COMPOSITION NUTRITIONNELLE POUR AMÉLIORER LE MICROBIOTE DE NOURRISSONS

(30) Priority: 03.12.2021 EP 21212356
(43) Date of publication of application: 09.10.2024
(73) Proprietor: N.V. Nutricia, 2712 HM Zoetermeer (NL)
(72) Inventor: VAN LIMPT, Cornelus Johannes Petrus, 3584 CT Utrecht (NL); GOUW, Joost Willem, 3584 CT Utrecht (NL); KNOL, Jan, 3584 CT Utrecht (NL); TIMS, Sebastian, 3584 CT Utrecht (NL); BEN AMOR, Kaouther, 3584 CT Utrecht (NL)
(74) Representative: Nederlandsch Octrooibureau
(86) International application number: PCT/EP2022/084244
(87) International publication number: WO 2023/099750

(56) References cited:
- WO-A1-2019/104390
- WO-A1-2021/078659
- US-A1- 2020 008 458
- BAKKER-ZIERIKZEE ASTRID MARIA: "Prebiotics and probiotics in infant nutrition", 19 January 2005 (2005-01-19), pages 1 - 152, XP055919912, Retrieved from the Internet <URL:https://edepot.wur.nl/34590>
- FABIANO VALENTINA ET AL: "Term Infant Formulas Influencing Gut Microbiota: An Overview", vol. 13, no. 12, 23 November 2021 (2021-11-23), pages 4200, XP055919904, Retrieved from the Internet <URL:https://www.ncbi.nlm.nih.gov/pmc/articles/PMC8708119/pdf/nutrients-13-04200.pdf> DOI: 10.3390/nu13124200
- SESTITO SIMONA ET AL: "The Role of Prebiotics and Probiotics in Prevention of Allergic Diseases in Infants", FRONTIERS IN PEDIATRICS, vol. 8, 22 December 2020 (2020-12-22), XP055919919, DOI: 10.3389/fped.2020.583946
- KONSTANTINOS C. MOUNTZOURIS ET AL: "Intestinal microflora of human infants and current trends for its nutritional modulation", BRITISH JOURNAL OF NUTRITION, vol. 87, no. 5, 1 May 2002 (2002-05-01), pages 405 - 420, XP055622794, ISSN: 0007-1145, DOI: 10.1079/BJN2002563

## Description

### FIELD OF THE INVENTION

The present invention is in the field of infant nutrition and relates to a nutritional composition for improving gut microbiota in infants or children particularly by increasing *Lactobacillus* and *Bifidobacterium* levels, said nutritional composition containing fructo-oligosaccharides and/or galactooligosaccharides in combination with beta-casein, wherein at least 75% by weight of the beta-casein is a beta-casein having a proline at position 67 of the beta-casein amino acid sequence.

### BACKGROUND OF THE INVENTION

The gastrointestinal microbiota is in close and continuous contact with epithelial and immune cells. This constant stimulation is essential for the development and functioning of the immune system. In addition, a healthy gut microbiota early in life has been associated with optimal cognitive development of infants and reduced chances of developing diseases later in life, such as obesity. Thus, the bacterial colonization of the gastrointestinal tract of a newborn is determinant for the healthy development of infants.

Microbial ecosystems are established during the first three years of life and form a symbiotic relationship with the host. The formation of the microbiological profile of the child's digestive tract is shaped by many factors, the most important of which are the type of delivery, feeding and environmental factors. It is known that children born by C-section develop a more adult-like microbiota and experience a delay in developing a balanced microbiota with abundance of beneficial species. Likewise, the microbiota of breast-fed babies is known to become typically dominated by bifidobacteria, whereas the microbiota of formula-fed babies tends to be more diverse with high numbers of Enterobacteriaceae, Enterococcaceae, Bifidobacteriaceae, Bacteroidaceae and Clostridiaceae.

In view of the health-promoting properties of bifidobacteria and lactobacilli, much attention should be paid to the population of these microorganisms in the gut of infants. Colonization of the intestinal microbiome during infancy represents a critical time for shaping gut health thereby optimizing positive immune effects and child's development.

Generally, the intestinal flora of human milk-fed infants is primarily composed of bifidobacteria and lactobacilli. Comparatively, the intestinal flora of formula-fed infants is more diverse and contains in general more *Bacteroides, Clostridium* and Enterobacteriaceae species. Formula-fed infants have about one-tenth to roughly two-third the number of bifidobacteria of human milk-fed infants. Bifidobacteria and lactobacilli are considered to be important in maintaining a well-balanced intestinal microbiota and it has been postulated that lactobacilli have several health-promoting effects, including the prevention and/or treatment of diarrhea and intestinal infections. Furthermore, lactobacilli have been shown to play a role in the immune system of the host. Early colonization also appears to be important for the establishment and maintenance of nonpathogenic intestinal microbiota.

Recent findings indicate that early colonization of the infant gastrointestinal tract with *Lactobacillus* is crucial for the overall health of infants, improvement of immune response and reduction of the incidence of several neonatal inflammatory conditions.

In particular, the presence of *lactobacilli* has been associated with lower levels of atopy-related chemokines during infancy, together with higher levels of interferon (IFN)-γ and lower FeNO during later childhood, indicating that lactobacilli species in the infant gut may influence allergy-related parameters in the peripheral immune system, and thereby contribute to allergy protection (Björkander, S et al. "Childhood allergy is preceded by an absence of gut lactobacilli species and higher levels of atopy-related plasma chemokines." Clinical and experimental immunology vol. 202,3 (2020): 288-299. doi:10.1111/cei.13494).

WO2021/078659 discloses a nutritional composition for a child, comprising galactooligosaccharides and A2 beta-casein, for use in improving the temperament of the child.

US8349386 relates to a proline-containing serum protein product, suitable as an ingredient for foods and therapeutic compositions, in particular infant and baby foods. The invention also provides a method for the preparation of the serum protein product, based on micro filtration of milk.

WO2019/104390 relates to a milk substitute composition comprising: bovine milk; additional protein, wherein the additional protein comprises whey and alpha-lactalbumin; additional fats, wherein the additional fats comprise sn-2 palmitate (as OPO triglycerides and/or sn-2 palmitate monoglyceride); prebiotic fibre; probiotic bacteria; wherein the bovine milk is substantially free from A1 beta-casein protein, wherein the milk substitute composition is suitable for administration to humans aged 0-36 months.

WO2018/063008 relates to milk and milk derived food products having a beta-casein composition that comprises predominantly A2 beta-casein or related beta-casein variants. The composition was associated with altered faecal levels of short-chain fatty acids in adults.

WO2021/003741A1 relates to A2 beta casein-enriched milk for adults, wherein the milk has been shown to promote proliferation of *Bifidobacterium* in adults.

### SUMMARY OF THE INVENTION

The inventors have surprisingly found that a composition comprising fructo-oligosaccharides and/or galactooligosaccharides in combination with beta-casein, wherein at least 75% by weight of the beta-casein is a beta-casein having a proline at position 67 of the beta-casein amino acid sequence synergistically increases *Lactobacillus* levels, presents a bifidogenic effect, and decreases the relative amounts of pathogenic bacteria thereby showing an advantageous effect on improving and modulating infant's or children's microbiota.

The inventors found that the components of the nutritional composition according to the invention act synergistically to promote the increase in *Lactobacillus* relative amounts. This is of particular benefit for infants and children whose gut microbiota is or is at risk of being susceptible to sub-optimal development, such as children born via C-section, preterm infants, exclusively or partially formula-fed infants, infants subject to antibiotic therapy, etc.

It is further hypothesized that the nutritional composition of the invention contributes to the overall healthy development of infants and children's microbiota and can be used in the prevention and/or treatment of disorders associated to an impaired gut microbiota, such as acute diarrhea, atopic diseases, antibiotic associated diarrhea, necrotizing enterocolitis, fine motor deficits, dyspraxia, allergies or combinations thereof.

### DRAWINGS

Figures 1 A-C show the results of Experiment 1 in Example 1, respectively for *Lactobacillus, Bifidobacterium* and *Escherichia-Shigella.*
Figure 2 shows the results of Experiment 2 in Example 1 for *Lactobacillus* growth.

### DETAILED DECSRIPTION OF THE INVENTION

The present invention thus concerns a nutritional composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence;
for use in improving the gut microbiota in an infant or child, wherein improvement of gut microbiota encompasses increasing *Lactobacillus* levels.

This aspect of the invention may also be worded as a method for improving the gut microbiota in an infant or child, wherein said improvement in the gut microbiota encompasses increasing *Lactobacillus* levels, said method comprising the administration of a nutritional composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence.

For some jurisdictions, this aspect of the invention may also be worded as the use of a composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence;
in the manufacture of a nutritional composition for improving the gut microbiota in an infant or child, wherein improvement of gut microbiota encompasses increasing *Lactobacillus* levels.

In yet a further aspect, the present invention concerns a nutritional composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence;
for use in treating and/or preventing conditions or disorders associated with impaired gut microbiota in an infant or child, wherein impairment of gut microbiota encompasses decreased *Lactobacillus* levels, and the conditions or disorders are selected from the group consisting of intestinal inflammation, infections originating from the intestine, and food allergy.

In case improvement of gut microbiota is considered non-therapeutic, the present invention can be worded as a non-therapeutic method of improving gut microbiota, wherein improving gut microbiota encompasses increasing *Lactobacillus* levels, the method comprising administering to an infant or child a composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence.

In the context of the present invention the term "prevention" means "reducing the risk of" or "reducing the severity of". The term "prevention of a certain condition" also includes "treatment of a person at risk of said condition". The methods or uses according to the present invention, comprising administering the present nutritional composition to a subject in need thereof, also refer to administering an effective amount of the nutritional composition to the subject.

### Beta-caseins

Beta-caseins of the nutritional composition of the invention are part of the total protein content.

The wt.% protein based on total dry weight of the present nutritional composition is calculated according to the Kjeldahl-method by measuring total nitrogen and using a conversion factor of 6.38 in case of casein, or a conversion factor of 6.25 for other proteins than casein. The term 'protein' or 'protein component' as used in the present invention refers to the sum of proteins, peptides and free amino acids.

Typically, the nutritional composition comprises 5-20 wt.% proteins, based on total dry weight of the composition, more preferably 8-15 wt.% proteins, even more preferably 9-12 wt.% proteins, based on total dry weight of the composition. Expressed differently, proteins preferably provide 6.5 to 20% of the total calories of the composition of the invention, preferably 9 to 16% of the total calories of the composition of the invention. For calculation of the % of total calories for the protein, the total of energy provided by proteins, peptides and amino acids needs to be taken into account. Preferably, proteins provide 1.6 to 4 g per 100 kcal, more preferably 1.7 to 2.3 g per 100 kcal of the nutritional composition.

Based on a ready-to-drink liquid product, proteins preferably provide 1 to 3 g per 100 ml, more preferably 1.1 to 2 g per 100 ml, even more preferably between 1.2 and 1.5 g, most preferably between 1.25 and 1.35 g per 100 ml.

Proteins used in the nutritional composition are preferably selected from the group consisting of non-human animal proteins, preferably milk proteins, vegetable proteins, and mixtures thereof. In one preferred embodiment, the composition includes 0-5 wt.% protein from vegetable source, based on total protein content, preferably 0-1 wt.% protein from vegetable source, more preferably 0 wt.% protein from a vegetable source.

The present nutritional composition preferably contains whey protein, more preferably bovine whey proteins. Thus in one preferred embodiment the protein in the present nutritional composition comprises protein selected from the group consisting of whey protein and casein, preferably from cow's milk. In case whey proteins are used, the protein source is preferably based on acid whey or sweet whey, whey protein isolate or mixtures thereof. The present nutritional composition preferably comprises casein and whey proteins in a weight ratio casein : whey protein of 10 : 90 to 90 : 10, more preferably 20 : 80 to 80 : 20, even more preferably 35 : 65 to 55 : 45.

Beta-caseins can be categorized as A1 beta-casein and A2 beta-casein. These two proteins are the predominant beta-caseins in the bovine milk consumed in most human populations. A1 beta-casein differs from A2 beta-casein by a single amino acid. A histidine amino acid is located at position 67 of the 209 amino acid sequence of A1 beta-casein, whereas a proline is located at the same position of A2 beta-casein. This single amino acid difference is, however, critically important to the enzymatic digestion of beta-caseins in the gut. The A1 beta-casein type is the most common type found in cow's milk in Europe and in the United States.

As used herein, a beta-casein variant that has a proline at position 67 of the beta-casein amino acid sequence preferably refers to A2 beta-casein. However, one skilled in the art will appreciate that it may be any A2 type beta-casein variant, i.e. including any but not limited to A2, A3, D and E beta-caseins which have proline at the homologous position of the beta-casein amino acid sequence.

The beta-casein having a proline at position 67 of the beta-casein amino acid sequence according to the invention is preferably isolated from milk of a non-human animal, more preferably bovine, caprine or ovine beta-casein. Most preferably, said beta-casein is bovine beta-casein.

Preferably the casein in the nutritional composition according to the invention is intact and/or non-hydrolyzed casein. In a preferred embodiment the beta-casein comprising at least 75wt% beta-casein having a proline at position 67 of the beta-casein amino acid sequence is intact and/or non-hydrolyzed.

The nutritional composition preferably comprises 0.3-7 wt.% beta-casein, based on dry weight of the composition, preferably 0.5-6 wt.% beta-casein, based on dry weight of the composition, more preferably 0.7-5 wt.% beta-casein, based on dry weight of the composition, most preferably 1-4 wt.% beta-casein, based on dry weight of the composition.

Preferably the amount of beta-casein present in the nutritional composition corresponds to 8-30 wt.%, more preferably 10-20 wt.%, most preferably 12-17 wt.% of the total protein content of said nutritional composition based on dry weight.

Preferably, at least 80 wt.%, more preferably at least 90 wt.%, even more preferably at least 98 wt.% of the beta-casein is a beta-casein variant that has a proline at position 67 of the beta-casein amino acid sequence nutritional composition. Most preferably, 100 wt.% of the beta-casein is a beta-casein variant that has a proline at position 67 of the beta-casein amino acid sequence nutritional composition.

According to one embodiment, the nutritional composition is substantially free of other types of beta-casein, preferably substantially free of A1 beta-casein. Herein, the term "substantially free of A1 beta-casein" means that the nutritional composition has an A1 beta-casein content of at least less than 5% w/w, for example less than 4%, less than 3%, less than 2%, less than 1%, or even less than 0.5% w/w A1 beta-casein protein. A1 beta-casein can be measured by any technique standard in the art of protein detection such as, for example, mass spectrometry, chromatography and electrophoresis.

### Non-digestible oligosaccharides

The nutritional composition comprises galactooligosaccharides (GOS), fructo-oligosaccharides (FOS), or combinations thereof. Galactooligosaccharides and fructo-oligosaccharides are non-digestible oligosaccharides and are fermented by the intestinal microbiota.

The present nutritional composition preferably comprises 1.75 to 17.5 wt.% total non-digestible oligosaccharides, more preferably 2.8 to 10.5 wt.%, most preferably 4.2 to 7 wt.%, based on total dry weight of the nutritional composition. Based on 100 ml, the present nutritional composition preferably comprises 0.25 to 2.5 g total non-digestible oligosaccharides, more preferably 0.4 to 1.5 g, most preferably 0.6 to 1 g, based on 100 ml of the nutritional composition. The total amount of non-digestible oligosaccharides includes galactooligosaccharides, fructo-oligosaccharides and any additional non-digestible oligosaccharides that may further be present in the composition.

Preferably, the nutritional composition comprises 80-2000 mg per 100 ml of fructo-oligosaccharides, galactooligosaccharides or combinations thereof, more preferably 100-1500 mg per 100 ml fructo-oligosaccharides, galactooligosaccharides or combinations thereof, most preferably 150-1000 mg per 100 ml fructo-oligosaccharides, galactooligosaccharides or combinations thereof, even more preferably 200-800 mg per 100 ml fructo-oligosaccharides, galactooligosaccharides or combinations thereof.

The nutritional composition according to the invention typically comprises 0.25-20 wt.% GOS and/or FOS based on total dry weight of the composition, more preferably 0.5-10 wt.% GOS and/or FOS based on total dry weight of the composition, most preferably 1.5-7.5 wt.% GOS and/or FOS based on total dry weight of the composition.

Preferably the nutritional composition according to the present invention comprises at least 0.3 g GOS and/or FOS per 100 kcal, more preferably at least 0.5 g, even more preferably at least 0.6 g per 100 kcal. Preferably the composition does not comprise more than 2.3 g of GOS and/or FOS per 100 kcal, preferably not more than 1.5 g per 100 kcal, more preferably not more than 1.2 g per 100 kcal. More preferably, the nutritional composition according to the present invention comprises GOS and/or FOS in an amount of 0.3 to 2.3 g per 100 kcal, even more preferably in an amount of 0.5 to 1.5 g per 100 kcal, even more preferably in an amount of 0.6 to 1.2 g per 100 kcal.

Preferred galactooligosaccharides according to the invention are transgalactooligosaccharides. Suitable galactooligosaccharides are for example Vivinal^{®}GOS (FrieslandCampina DOMO).

Preferred fructo-oligosaccharides (FOS) according to the invention are long chain FOS. The term fructo-oligosaccharides as used in the present invention refers to carbohydrates composed of over 50%, preferably over 65 % fructose units based on monomeric subunits, in which at least 50%, more preferably at least 75%, even more preferably at least 90%, of the fructose units are linked together via a beta-glycosidic linkage, preferably a beta-2,1 glycosidic linkage. A glucose unit may be present at the reducing end of the chain of fructose units. Preferably the fructo-oligosaccharides have a DP or average DP in the range of 2 to 250, more preferably 2 to 100, even more preferably 10 to 60. The term fructo-oligosaccharides comprises levan, hydrolysed levan, inulin, hydrolysed inulin, and synthesized fructo-oligosaccharides. Preferably the preparation comprises long chain fructo-oligosaccharides with an average DP above 20. Fructo-oligosaccharide suitable for use in the composition of the invention is also readily commercially available, e.g. RaftilineHP (Orafti).

Preferably the nutritional composition according to the present invention comprises at least 25 mg FOS per 100 ml, more preferably at least 40 mg, even more preferably at least 60 mg per 100 ml. Preferably the nutritional composition does not comprise more than 250 mg FOS per 100 ml, more preferably not more than 150 mg per 100 ml and most preferably not more than 100 mg FOS per 100 ml. The amount of FOS is preferably 25 to 250 mg fructo-oligosaccharides per 100 ml, more preferably 40 to 150 mg per 100 ml, even more preferably 60 to 100 mg per 100 ml. Preferably the nutritional composition according to the present invention comprises at least 0.15 wt.% FOS based on total dry weight, more preferably at least 0.25 wt.%, even more preferably at least 0.4 wt.%. Preferably the composition does not comprise more than 1.5 wt.% FOS based on total dry weight of the composition, more preferably not more than 1.0 wt.%. The presence of FOS shows a further improved effect on the microbiota and its SCFA production when combined with the galactooligosaccharides.

Preferably the mixture of galactooligosaccharides and fructo-oligosaccharides is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when the galactooligosaccharides have a low average DP and fructo-oligosaccharides has a relatively high DP. Most preferred is a mixture of galactooligosaccharides with an average DP below 10, preferably below 6, and fructo-oligosaccharides with an average DP above 7, preferably above 11, even more preferably above 20. In a preferred embodiment, the nutritional composition comprises a mixture of transgalactooligosaccharides and long chain fructo-oligosaccharides.

### Application

The nutritional composition according to the invention advantageously improves the gut microbiota of infants and/or children, preferably infants aged 0-36 months, wherein improvement of gut microbiota encompasses increasing *Lactobacillus* levels.

Increasing the *Lactobacillus* levels is herein defined as increasing the relative *Lactobacillus* count in the infant's or children's feces and/or regulating the *Lactobacillus* population in the infant's or children's intestinal tract on a species level as compared to levels seen when the infant or child is fed with a nutritional composition comprising beta-casein wherein at least 75% by weight of the beta-casein is a beta-casein variant that has a histidine amino acid at position 67 of the beta-casein amino acid sequence (*i.e.*, A1 beta-casein).

The nutritional composition is preferably for use in improving the gut microbiota, wherein said improvement encompasses increasing *Lactobacillus* levels in the large intestine. The terms *"Lactobacillus"* and "lactobacilli" as used herein are used interchangeably and have equal meaning.

Preferably, the improvement of gut microbiota further encompasses increasing the amounts of *Bifidobacterium* and/or decreasing the amounts of pathogenic bacteria. The terms *"Bifidobacterium"* and "bifidobacteria" as used herein are used interchangeably and have equal meaning.

More preferably, the improvement of gut microbiota further encompasses at least one of: increasing *Bifidobacterium* levels and/or decreasing the pathogenic bacteria levels in the infant's or children's feces and/or in the infant's or children's intestinal tract on a species level as compared to levels seen when the infant of child is fed with a nutritional composition comprising beta-casein wherein at least 75% by weight of the beta-casein is a beta-casein variant that has a histidine amino acid at position 67 of the beta-casein amino acid sequence (i.e., A1 beta-casein).

Preferably, the pathogenic bacteria are selected from the family of Enterobacteriaceae, Veillonellaceae, Clostridiaceae, Enterococcaceae, or combinations thereof. The family Veillonellaceae may be associated with Crohn's disease in children, and C-section born infants. More preferably, the pathogenic bacteria are Enterobacteriaceae, and most preferably the pathogenic bacteria are *Escherichia, Shigella* or combinations thereof.

'Infants' as used herein broadly refer to infants and young children from 0-36 months. Preferably, the composition is for an infant aged between 0-24 months, more preferably 0-12 months, most preferably between 0-6 months.

'Children' as used herein broadly refers to kids aged from 3 years old to 12 years old.

Preferably, the methods or uses according to the invention are for infants and young children.

In a preferred embodiment, the methods or uses according to the present invention are for healthy infants, preferably for healthy, term infants. In newborns, the intestinal tract is not yet fully developed and infants are in need of support for developing a healthy, balanced gut microbiota thereby developing resistance to pathogens and/or allergens.

In a preferred embodiment the nutritional composition is administered to an infant or child having or at risk of having a compromised microbiota, i.e., an unhealthy and/or sub-optimal microbiota profile. An unhealthy and/or sub-optimal microbiota profile means increased presence of pathogenic bacteria and/or decreased presence of beneficial bacteria. In infants, this is in comparison to the microbiota of healthy infants, preferably naturally born and breastfed.

In infants, impaired, unbalanced (dysbiosis) or sub-optimal gut microbiota can be easily determined without invasive techniques, i.e., using state of the art microbiology techniques for analysing fecal samples.

Preferably, the infant is selected from the group of infants born via cesarean section, preterm infants, infants born from a mother who received intrapartum antibiotics, infants or children receiving or having received antibiotic therapy, formula fed infants, including exclusively formula fed infants, or combinations thereof.

The aforementioned nutritional composition may be administered to infants or children to prevent and/or treat acute diarrhea, atopic diseases, antibiotic associated diarrhea, necrotizing enterocolitis, fine motor deficits, dyspraxia, allergies or combinations thereof, in infants in need thereof.

In an embodiment of the present invention, the nutritional composition for use, or the nutritional composition in the methods and uses according to the invention is administered on two days or more per week, for example on three days or four days or five days or six days per week or daily.

### Nutritional composition

The nutritional composition of the invention is not human milk, native cow's milk or native milk from another mammal.

In a preferred embodiment, the nutritional composition according to the invention is an infant formula, follow on formula, young child formula, kids supplement, more preferably an infant formula. The terms "infant formula" or "follow-on formula" or "young child formula" or "kids supplement" as used herein mean that the composition is artificially made or in other words that it is synthetic. In the context of the present invention, an infant formula and follow-on formula is defined for a human having an age of 0 to 12 months and a young child formula is defined for a human having an age of 13 to 36 months. Kids supplement is herein defined as a composition for children above 3 years of age, preferably from 3 to 12 years of age.

The nutritional composition according to the invention may comprise probiotic bacteria. The probiotic bacteria content of the nutritional composition is preferably in the range 10⁴- 10¹¹ cfu/100g, more preferably 10⁷ -10¹⁰cfu/100g.

In a preferred embodiment, said probiotic bacteria is selected from *Bifidobacterium* and/or *Lactobacillus,* more preferably the probiotic bacteria is selected from infant type *Bifidobacterium* strains, even more preferably the probiotic bacteria is selected from *Bifidobacterium breve, Bifidobacterium longum* subsp. *infantis, Bifidobacterium longum* subsp. *longum, Bifidobacterium bifidum* and combinations thereof. Preferably, the nutritional composition comprises *Bifidobacterium breve,* more preferably *Bifidobacterium breve* M-16V. According to another embodiment of the invention, the nutritional composition is free of probiotics.

In a preferred embodiment the nutritional composition is a powder. Preferably, the powder is reconstituted with aqueous liquid, such as water or milk, to prepare a liquid composition. Preferably, the aqueous liquid is water. Preferably, the reconstituted liquid composition has a pH between 6 and 8, more preferably between 6.5 and 7.5. The nutritional composition is preferably administered orally to the infant or child.

The present nutritional composition preferably comprises digestible carbohydrate providing 5 to 20 g per 100 kcal, preferably 8 to 15 g per 100 kcal. Preferably the amount of digestible carbohydrate in the present nutritional composition is 25 to 90 wt.%, based on total dry weight of the composition. Preferred digestible carbohydrates are lactose, glucose, sucrose, fructose, galactose, maltose, starch and maltodextrin. Lactose is the main digestible carbohydrate present in human milk. The present nutritional composition preferably comprises lactose. Preferably the present nutritional composition does not comprise high amounts of carbohydrates other than lactose. Compared to digestible carbohydrates such as maltodextrin, sucrose, glucose, maltose and other digestible carbohydrates with a high glycemic index, lactose has a lower glycemic index and is therefore preferred. The present nutritional composition preferably comprises digestible carbohydrate, wherein at least 35 40 wt.%, more preferably at least 50 wt.%, more preferably at least 60 wt.%, more preferably at least 75 wt.%, even more preferably at least 90 wt.%, most preferably at least 95 wt.% of the digestible carbohydrate is lactose. Based on total dry weight the present nutritional composition preferably comprises at least 25 wt.% lactose, preferably at least 40 wt.%, more preferably at least 50 wt.% lactose.

The nutritional composition may comprise lipids. Lipid in the present invention comprises one or more selected from the group consisting of triglycerides, polar lipids (such as phospholipids, cholesterol, glycolipids, sphingomyelin), free fatty acids, monoglycerides and diglycerides. The lipid provides preferably 30 to 60 % of the total calories of the nutritional composition. More preferably the nutritional composition comprises lipid providing 35 to 55 % of the total calories, even more preferably the nutritional composition comprises lipids providing 40 to 50 % of the total calories. The lipids are preferably present in an amount of 3 to 7 g per 100 kcal, more preferably in an amount of 4 to 6 g lipid per 100 kcal and most preferably in an amount of 4.5 to 5.5 g lipid per 100 kcal. When in liquid form, e.g. as a ready-to-feed liquid, the nutritional composition preferably comprises 2.1 to 6.5 g lipids per 100 ml, more preferably 3.0 to 4.0 g per 100 ml. Based on dry weight the nutritional composition preferably comprises 10 to 50 wt.%, more preferably 12.5 to 40 wt.% lipids, even more preferably 19 to 30 wt.% lipids.

The lipid preferably comprises vegetable lipids. The presence of vegetable lipids advantageously enables an optimal fatty acid profile high in polyunsaturated fatty acids and/or more reminiscent to human milk fat. Lipid from non-human mammalian milk alone, e.g. cow's milk, does not provide an optimal fatty acid profile. The amount of essential fatty acids is too low in non-human mammalian milk.

Preferably the nutritional composition comprises at least one, preferably at least two vegetable lipid sources selected from the group consisting of linseed oil (flaxseed oil), rape seed oil (such as colza oil, low erucic acid rape seed oil and canola oil), sunflower oil, high oleic sunflower oil, safflower oil, high oleic safflower oil, olive oil, coconut oil, palm oil and palm kernel oil.

In a preferred embodiment, the nutritional composition comprises 5 to 100 wt.% vegetable lipids based on total lipids, more preferably 10 to 95 wt.%, more preferably 20 to 80 wt.%, even more preferably 25 to 75 wt.%, most preferably 40 to 70 wt.% vegetable lipids based on total lipids. It is noted therefore that the nutritional composition also may comprise non-vegetable lipids. Non-vegetable lipids may include mammalian milk fat, mammalian milk derived lipid as a preferred source of phospholipid, and fish, marine and/or microbial oils as source of long chain polyunsaturated fatty acids (LC-PUFA).

The nutritional composition is preferably an infant formula or follow-on formula and preferably comprises 3 to 7 g lipid/100 kcal, more preferably 4 to 6 g lipid/100 kcal, even more preferably 4.5 to 5.5 g lipid/100 kcal, preferably comprises 1.7 to 3.5 g protein/100 kcal, more preferably 1.8 to 2.1 g protein/100 kcal, even more preferably 1.8 to 2.0 g protein/100 kcal and preferably comprises 5 to 20 g digestible carbohydrate/100kcal, more preferably 6 to 16 g digestible carbohydrate/100 kcal, even more preferably 10 to 15 g digestible carbohydrate/100 kcal. Preferably the nutritional composition is an infant formula or follow-on formula and has an energy density of 60 kcal to 75 kcal/100 ml, more preferably 60 to 70 kcal/100 ml, when in a ready-to-drink form. This density ensures an optimal balance between hydration and caloric intake.

The nutritional composition according to the present invention does not have an excessive caloric density, however still provides sufficient calories to feed the subject. Hence, the liquid food preferably has a caloric density between 0.1 and 2.5 kcal/ml, more preferably a caloric density of between 0.5 and 1.5 kcal/ml, even more preferably between 0.6 and 0.8 kcal/ml, and most preferably between 0.65 and 0.7 kcal/ml.

The invention is further illustrated by the following non-limiting examples.

### EXAMPLES

### Example 1 - Gut microbiota modulation

The effects of the combination of A2 beta-casein with oligosaccharides on the gut microbiota were studied using a simulated digested composition with A2 beta-casein hydrolysate ('A2 digest') in combination with oligosaccharides.

### Methods

'A2 digest' was prepared by defatting 40 mL of A2 milk (commercially available from supermarket) by 20 minutes centrifugation at 4°C and 5000 rpm. The upper fat layer was discarded and the milk was defatted again. The casein proteins were precipitated by adjusting the pH to 4.6 followed by centrifugation for 15 minutes at 2000x g. The liquid layer above the pellet was discarded and 10 mL of 50 mM ammonium bicarbonate was added to the pellet. Food-grade trypsin (1:50) was used to digest the proteins overnight at 37 °C. Formic acid and heat (10 minutes at 95 °C) were used to quench the remaining trypsin activity. The digested proteins were freeze dried for approximately 24 hours.

Fecal samples from a formula-fed infant (Nutrilon 2, containing A1-beta-casein and GOS/FOS) were prepared under anaerobic conditions at 10% w/v dilution in 2x concentrated infant adapted Colonic Microbiota Medium (CMM) with or without 2.5 g/L mucus. (CMM composition: Yeast extract 1 gL, Ammoniumsulphate 2 g L, K₂HPO₄ 2 g·L, NaHCO₃ 3.2 g L, NaCl 4,5 g L, MgSO₄.7H₂O 0.5 g L, Cystein HCl 0.5 g L, CaCl₂.2H₂O 0.4 g L, Bile Salts 25 mg L mendione, metal and vitamin solution, heamin (10 mg/L), Mucus 2.5 g.L., pH of medium was adjusted to pH 5.8).

The samples were filtered through a tea sieve followed by 100 µm filtration (steriflip). One ml of the fecal slurry was added to all 32 fermenter wells of a 48 Wells Microfluidic Round Well Plate (MTP-RMF32-BOH2, M2P Labs Baeseweiler, Germany) with feeding module and pH control. 200 µl of the 10x concentrated carbohydrate solutions or water (see table A), 200 µl of 10x A2 casein digest, 200 µl of sterile 10x mucus (porcine gastric) solution or water and 360 µl water + 40 µl *B. breve* M-16V or 400 µl of sterile water was added to the respective cells according to the experimental template. The feeding wells were filled with 10x carbohydrate solutions for automatic feeding during nighttime (manual feeding during daytime) according to template. The pH control wells were added with 3M NaOH. The pH was during the entire experiment controlled at pH 5.8. The MTP plate was then sealed with silicone foil with filtered venting holes and placed into the BioLector Pro (M2P Labs Baeseweiler, Germany). 4 Hours after fermentation (anaerobic, 4 hrs, 800 RPM, 37°C, 85% humidity, pH control at 5.8) started, the content of the wells was harvested and centrifuged for 1 minute at 16.400 x g. The biomass (pellet) was resuspended again in 2xCMM and returned in the same fermenter. Supernatant was frozen and used for further analyses.

To the fermenter wells the carbohydrates, A2 digest, mucus and water were added as described before and run was started again. This procedure was repeated for 3 days. In total eight feedings over 56 hours were performed to see the effect of the interventions on metabolic activity and microbiota composition. All conditions were performed in fourfold. Fecal supernatants of the fecal fermentations were thawed and aliquoted to quantitatively determine SCFA acetic, propionic, n-butyric, isovaleric and n-valeric acids by gas chromatography, ammonia concentration by the rapid Ammonia kit (Megazyme) and after heat inactivation D- and L-lactate by the D-lactic acid/L-lactic acid kit (R-Biopharm AG).

Statistics was done by using the statistical program GraphPad Prism, version 8.5. One-way ANOVA for multiple comparisons, depending if data where normally distributed parametric or non- parametric, was used. Post-hoc testing was then respectively done by Tuckey or Dunn's method. P-Values, after post-hoc testing, smaller than 0.05 were considered significant. Fecal pellets from starting and end of fermentation were sent for 16S rRNA sequencing.

The tested groups are described in Table A.

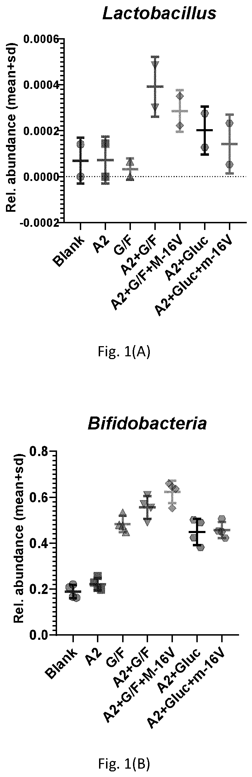

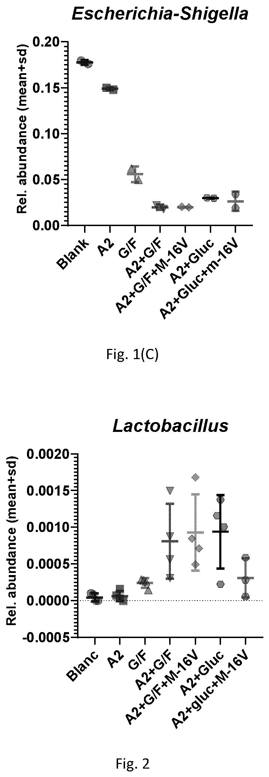

The results of Experiment 1 are shown in Figures 1A (*Lactobacillus*), 1B (*Bifidobacterium*) and 1C (*Escherichia-Shigella*). The results of Experiment 2 for *Lactobacillus* growth is shown in Fig. 2.

The combination of A2 beta-casein with oligosaccharides synergistically increased the *Lactobacillus* count in the samples. Advantageously, the amounts of *Bifidobacterium* are favored, whereas growth of opportunistic pathogenic *Escherichia-Shigella* species are inhibited with the composition according to the invention.

**Table A - Tested groups at final concentrations in fermenter**

| Tested groups* | Experiment 1 | Experiment 2 |
|---|---|---|
| | +/- Mucus (0.25%) | + Mucus (0.25%) |
| (1) Water (blank) | No additional Carbohydrates | No additional Carbohydrates |
| | No additional protein/Nitrogen | No additional protein/Nitrogen |
| (2) GOS+FOS** | 1% (w/v) total carbohydrates | 0.5% (w/v) total carbohydrates |
| | No additional protein/Nitrogen | No additional protein/Nitrogen |
| (3) Glucose | 1% (w/v) total carbohydrates | 0.5% (w/v) total carbohydrates |
| | No additional protein/Nitrogen | No additional protein/Nitrogen |
| (4) GOS+FOS** + A2 protein digest | 1% (w/v) total carbohydrates | 0.5% (w/v) total carbohydrates |
| | 2.5 gram/L A2 digest | 2.5 gram/L A2 digest |
| (5) Glucose + A2 protein digest | 1% (w/v) total carbohydrates | 0.5% (w/v) total carbohydrates |
| | 2.5 gram/L A2 digest | 2.5 gram/L A2 digest |
| (6) GOS+FOS** + A2 protein digest *+ B. breve**** | 1% (w/v) total carbohydrates | 0.5% (w/v) total carbohydrates |
| | 2.5 gram/L A2 digest | 2.5 gram/L A2 digest |
| | *Bifidobacterium breve* M-16V (1x10E9 CFU/ml) | *Bifidobacterium breve* M-16V (1x10E9 CFU/ml) |
| (7) Glucose + A2 protein digest *+ B. breve**** | 1% (w/v) total carbohydrates | 0.5% (w/v) total carbohydrates |
| | 2.5 gram/L A2 digest | 2.5 gram/L A2 digest |
| | *Bifidobacterium breve* M-16V (1x10E9 CFU/ml) | *Bifidobacterium breve* M-16V (1x10E9 CFU/ml) |

| | | |
|---|---|---|
| * Colonic microbiota medium (CMM) ** Short Chain Galacto-oligosaccharides (Vivinal): Fructo-oligosaccharides HP 9:1 *** Probiotic strain: *Bifidobacterium breve* M-16V *B.breve* M-16V | | |

## Claims

1. A nutritional composition comprising:
- galactooligosaccharides, fructo-oligosaccharides, or combinations thereof and
- beta-casein, wherein at least 75% by weight of the beta-casein is a beta-casein variant that has a proline at position 67 of the beta-casein amino acid sequence;
for use in improving gut microbiota in an infant or a child, wherein improvement of gut microbiota encompasses increasing *Lactobacillus* levels.

2. The nutritional composition for use according to claim 1, wherein improvement of gut microbiota further encompasses increasing the amounts of *Bifidobacterium,* and/or decreasing the amounts of pathogenic bacteria.

3. The nutritional composition for use according to claim 1 or 2, wherein the nutritional composition comprises 0.25-20 wt.% galactooligosaccharides, fructo-oligosaccharides, or combinations thereof, based on dry weight of the composition.

4. The nutritional composition for use according to any one of the preceding claims, wherein the nutritional composition comprises 0.3-7 wt.% beta-casein, based on dry weight of the composition.

5. The nutritional composition for use according to any one of the preceding claims, wherein at least 80 wt.% of the beta-casein is a beta-casein variant that has a proline at position 67 of the beta-casein amino acid sequence nutritional composition.

6. The nutritional composition for use according to any one of the preceding claims, wherein the nutritional composition is substantially free of A1 beta-casein.

7. The nutritional composition for use according to any one of the preceding claims, wherein the nutritional composition further comprises *Bifidobacterium breve,* preferably *Bifidobacterium breve* M16V.

8. The nutritional composition for use according to any one of the preceding claims, wherein the infant or child has or is at risk of having a compromised microbiota.

9. The nutritional composition for use according to claim 8, wherein the infant or child is selected from the group of infants born via cesarean section, preterm infants, infants born from a mother who received intrapartum antibiotics, infants or children receiving or having received antibiotic therapy, formula fed infants, or combinations thereof.

10. The nutritional composition for use according to any one of the preceding claims, wherein the infant is aged between 0-24 months.

11. The nutritional composition according to any one of the preceding claims, wherein the composition is orally administered to the infant or child.

12. The nutritional composition for use according to any one of the preceding claims, wherein the composition is a powder.

13. The nutritional composition for use according to any one of the preceding claims, wherein the composition is an infant formula, a follow on formula, a young child formula or a kids supplement.

14. The nutritional composition for use according to any one of the preceding claims, wherein improvement of gut microbiota includes decreasing the amounts of pathogenic bacteria, wherein the pathogenic bacteria are selected from the family of Enterobacteriaceae, Veillonellaceae, Clostridiaceae, Enterococcaceae, or combinations thereof.

15. The nutritional composition for use according to claim 14, wherein the pathogenic bacteria are Enterobacteriaceae, preferably the pathogenic bacteria are *Escherichia, Shigella* or combinations thereof.

## Patentansprüche

1. Nährstoffzusammensetzung, umfassend:
- Galactooligosaccharide, Fructooligosaccharide oder Kombinationen davon und
- Beta-Casein, wobei mindestens 75 Gew.-% des Beta-Caseins eine Beta-Casein-Variante ist, die an Position 67 der Beta-Casein-Aminosäuresequenz ein Prolin aufweist;
zur Anwendung zur Verbesserung der Darmmikrobiota bei einem Säugling oder einem Kind, wobei die Verbesserung der Darmmikrobiota eine Erhöhung der Lactobacillus-Spiegel umfasst.

2. Nährstoffzusammensetzung zur Anwendung nach Anspruch 1, wobei die Verbesserung der Darmmikrobiota weiter die Erhöhung der Mengen an *Bifidobacterium* und/oder die Verringerung der Mengen an pathogenen Bakterien umfasst.

3. Nährstoffzusammensetzung zur Anwendung nach Anspruch 1 oder 2, wobei die Nährstoffzusammensetzung 0,25-20 Gew.-% Galactooligosaccharide, Fructooligosaccharide oder Kombinationen davon umfasst, basierend auf dem Trockengewicht der Zusammensetzung.

4. Nährstoffzusammensetzung zur Anwendung nach einem der voranstehenden Ansprüche, wobei die Nährstoffzusammensetzung 0,3-7 Gew.-% Beta-Casein, basierend auf dem Trockengewicht der Zusammensetzung, umfasst.

5. Nährstoffzusammensetzung zur Anwendung nach einem der voranstehenden Ansprüche, wobei mindestens 80 Gew.-% des Beta-Caseins eine Beta-Casein-Variante ist, die ein Prolin an Position 67 der Beta-Casein-Aminosäuresequenz der Nährstoffzusammensetzung aufweist.

6. Nährstoffzusammensetzung zur Anwendung nach einem der voranstehenden Ansprüche, wobei die Nährstoffzusammensetzung im Wesentlichen frei von A1-Beta-Casein ist.

7. Nährstoffzusammensetzung zur Anwendung nach einem der voranstehenden Ansprüche, wobei die Nährstoffzusammensetzung weiter *Bifidobacterium breve,* vorzugsweise *Bifidobacterium breve* M16V, umfasst.

8. Nährstoffzusammensetzung zur Anwendung nach einem der voranstehenden Ansprüche, wobei der Säugling oder das Kind eine beeinträchtigte Mikrobiota aufweist oder ein Risiko dafür hat.

9. Nährstoffzusammensetzung zur Anwendung nach Anspruch 8, wobei der Säugling oder das Kind ausgewählt ist aus der Gruppe von Säuglingen, die per Kaiserschnitt geboren wurden, Frühgeborenen, Säuglingen, die von einer Mutter geboren wurden, die intrapartale Antibiotika erhalten hat, Säuglingen oder Kindern, die eine Antibiotikatherapie erhalten oder erhalten haben, Säuglingen, die mit Säuglingsanfangsnahrung ernährt werden oder Kombinationen davon.

10. Nährstoffzusammensetzung zur Anwendung nach einem der voranstehenden Ansprüche, wobei der Säugling ein Alter zwischen 0-24 Monaten aufweist.

11. Nährstoffzusammensetzung nach einem der voranstehenden Ansprüche, wobei die Zusammensetzung dem Säugling oder Kind oral verabreicht wird.

12. Nährstoffzusammensetzung zur Anwendung nach einem der voranstehenden Ansprüche, wobei die Zusammensetzung ein Pulver ist.

13. Nährstoffzusammensetzung zur Anwendung nach einem der voranstehenden Ansprüche, wobei die Zusammensetzung eine Säuglingsanfangsnahrung, eine Folgenahrung, eine Kleinkindnahrung oder ein Nahrungsergänzungsmittel für Kinder ist.

14. Nährstoffzusammensetzung zur Anwendung nach einem der voranstehenden Ansprüche, wobei die Verbesserung der Darmmikrobiota eine Verringerung der Mengen an pathogenen Bakterien einschließt, wobei die pathogenen Bakterien aus der Familie der Enterobacteriaceae, Veillonellaceae, Clostridiaceae, Enterococcaceae oder Kombinationen davon ausgewählt sind.

15. Nährstoffzusammensetzung zur Anwendung nach Anspruch 14, wobei die pathogenen Bakterien Enterobacteriaceae sind, wobei die pathogenen Bakterien vorzugsweise *Escherichia, Shigella* oder Kombinationen dieser sind.

## Revendications

1. Composition nutritionnelle comprenant :
- des galactooligosaccharides, des fructo-oligosaccharides ou des combinaisons de ceux-ci, et
- de la bêta-caséine, où au moins 75 % en poids de la bêta-caséine est une variante de bêta-caséine qui comporte une proline en position 67 de la séquence d'acides aminés de la bêta-caséine ;
pour utilisation pour l'amélioration du microbiote intestinal chez un nourrisson ou un enfant, où l'amélioration du microbiote intestinal comprend l'augmentation des niveaux de *Lactobacillus.*

2. Composition nutritionnelle pour utilisation selon la revendication 1, où l'amélioration du microbiote intestinal comprend en outre l'augmentation des quantités de *Bifidobacterium* et/ou la diminution des quantités de bactéries pathogènes.

3. Composition nutritionnelle pour utilisation selon la revendication 1 ou 2, où la composition nutritionnelle comprend 0,25 à 20 % en poids de galactooligosaccharides, de fructo-oligosaccharides ou de combinaisons de ceux-ci, sur la base du poids sec de la composition.

4. Composition nutritionnelle pour utilisation selon l'une quelconque des revendications précédentes, où la composition nutritionnelle comprend 0,3 à 7 % en poids de bêta-caséine, sur la base du poids sec de la composition.

5. Composition nutritionnelle pour utilisation selon l'une quelconque des revendications précédentes, où au moins 80 % en poids de la bêta-caséine est une variante de bêta-caséine qui comporte une proline en position 67 de la séquence d'acides aminés de la bêta-caséine de la composition nutritionnelle.

6. Composition nutritionnelle pour utilisation selon l'une quelconque des revendications précédentes, où la composition nutritionnelle est pratiquement exempte de bêta-caséine A1.

7. Composition nutritionnelle pour utilisation selon l'une quelconque des revendications précédentes, où la composition nutritionnelle comprend en outre *Bifidobacterium breve,* de préférence *Bifidobacterium breve* M16V.

8. Composition nutritionnelle pour utilisation selon l'une quelconque des revendications précédentes, où le nourrisson ou l'enfant présente ou risque de présenter un microbiote compromis.

9. Composition nutritionnelle pour utilisation selon la revendication 8, où le nourrisson ou l'enfant est choisi parmi le groupe des nourrissons nés par césarienne, des nourrissons prématurés, des nourrissons nés d'une mère ayant reçu des antibiotiques intra-partum, des nourrissons ou des enfants recevant ou ayant reçu un traitement antibiotique, des nourrissons nourris à la préparation pour nourrissons, ou des combinaisons de ceux-ci.

10. Composition nutritionnelle pour utilisation selon l'une quelconque des revendications précédentes, où le nourrisson est âgé de 0 à 24 mois.

11. Composition nutritionnelle selon l'une quelconque des revendications précédentes, où la composition est administrée par voie orale au nourrisson ou à l'enfant.

12. Composition nutritionnelle pour utilisation selon l'une quelconque des revendications précédentes, où la composition est une poudre.

13. Composition nutritionnelle pour utilisation selon l'une quelconque des revendications précédentes, où la composition est une préparation pour nourrissons, une préparation de suite, une préparation pour jeunes enfants ou un complément alimentaire pour enfants.

14. Composition nutritionnelle pour utilisation selon l'une quelconque des revendications précédentes, où l'amélioration du microbiote intestinal inclut la diminution des quantités de bactéries pathogènes, où les bactéries pathogènes sont choisies parmi la famille des Enterobacteriaceae, des Veillonellaceae, des Clostridiaceae, des Enterococcaceae, ou des combinaisons de celles-ci.

15. Composition nutritionnelle pour utilisation selon la revendication 14, où les bactéries pathogènes sont des Enterobacteriaceae, de préférence les bactéries pathogènes sont *Escherichia, Shigella* ou des combinaisons de celles-ci.
